# EUROPÄISCHE PATENTSCHRIFT

(11) **EP 3 541 469 B1**
(45) Veröffentlichungstag und Bekanntmachung des Hinweises auf die Patenterteilung: **23.12.2020**
(21) Anmeldenummer: 17808823.3
(22) Anmeldetag: 17.11.2017
(51) Int. Cl.: A61N 1/05, A61N 1/36, A61B 5/04

(54) **IMPLANTIERBARE ELEKTRISCHE, MULTIPOLIGE VERBINDUNGSSTRUKTUR**
IMPLANTABLE ELECTRIC MULTI-POLE CONNECTION STRUCTURE
STRUCTURE DE CONNEXION MULTIPOLAIRE ÉLECTRIQUE IMPLANTABLE

(30) Priorität: 18.11.2016 DE 102016222712
(43) Veröffentlichungstag der Anmeldung: 25.09.2019
(73) Patentinhaber: Neuroloop GmbH, 79108 Freiburg (DE)
(72) Erfinder: KIMMIG, Fabian, 79110 Freiburg (DE); BORETIUS, Tim, 79108 Freiburg (DE); PLACHTA, Dennis, 79279 Vörstetten (DE)
(74) Vertreter: Rösler, Uwe
(86) Internationale Anmeldenummer: PCT/EP2017/079593
(87) Internationale Veröffentlichungsnummer: WO 2018/091654

(56) Entgegenhaltungen:
- DE-A1-102014 014 927
- US-A- 5 324 322
- US-A1- 2008 082 137
- US-A1- 2010 192 374
- US-A1- 2011 077 699

## Beschreibung

### Technisches Gebiet

Die Erfindung bezieht sich auf eine implantierbare elektrische, multipolige Verbindungsstruktur zwischen einem elektrischen Implantat und einer elektrischen Zu- und Ableitungsstruktur.

### Stand der Technik

Elektronische Implantate, die für einen dauerhaften oder zumindest langfristigen Verbleib im Körper geeignet sind, werden typischerweise zur therapeutischen Beeinflussung von Organfunktionen eingesetzt. Bekannte Beispiele sind Herz- oder Hirnschrittmacher. Gattungsgemäße Implantate verfügen je nach Anwendungszweck und Komplexität der therapeutischen Zielsetzung über eine Vielzahl elektrischer Zu- und Ableitungen, über das jeweilige Implantat mit elektrischen Steuer- bzw. Regelsignalen sowie mit elektrischer Energie versorgt wird. Die Anzahl der elektrischen Zu- bzw. Ableitungen bei elektronisch komplexen Implantaten kann durchaus zwanzig und mehr elektrische Leitungen umfassen, die zu einem flexiblen Kabel zusammengefasst sind, über das das Implantat mit einem Steuergerät, das zumeist mit einer Energiequelle kombiniert ist, verbindet. Die intrakorporale Verortung des Steuergerätes sowie der Energiequelle erfolgt zumeist subkutan in einem Körperbereich, wie beispielsweise im Brustbereich oder nahe des Schlüsselbeins, in dem für die Person möglichst geringe bewegungsbedingte äußere sowie innere Belastungen auftreten und ein leichter operativer Zugang möglich ist.

In der Regel sind die elektrischen Zu- und Ableitungen zwischen dem Implantat und dem Steuergerät bzw. der Energiequelle nicht einstückig ausgebildet, sondern über wenigstens eine Schnittstelle in Form einer intrakorporalen Steckverbindung oder einer lös- oder unlösbaren elektrischen Verbindung, wie beispielsweise als Bond- oder Lötverbindung, realisiert. Dies wirft zum einen Probleme auf, die mit dem für die Schnittstelle erforderlichen Bauraum zusammenhängen, darüber hinaus ist es erforderlich, den Schnittstellenbereich aufgrund des feuchten intrakorporalen Milieus entsprechend feuchteresistent auszubilden.

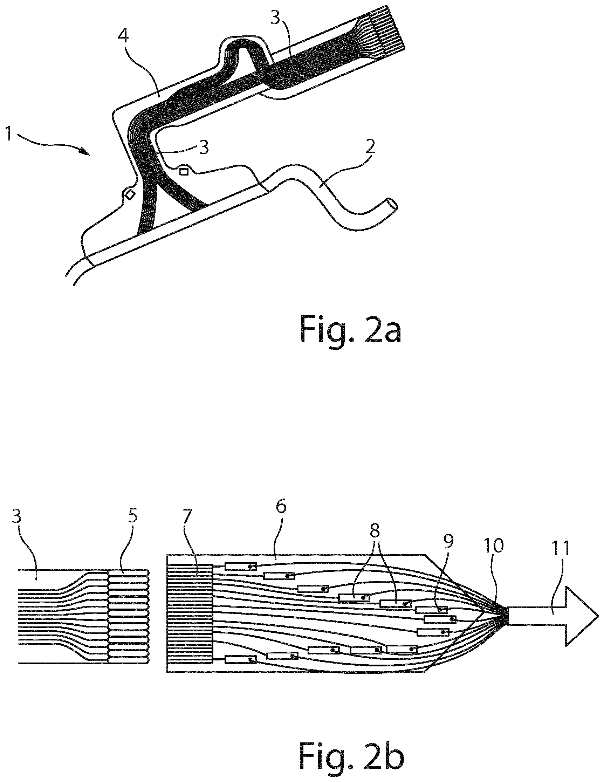

Am Beispiel einer über eine vielpolige Zu- und Ableitungsstruktur zu versorgende, implantierbare Cuff-Elektrodenanordnung soll die bislang bestehende Problematik einer an sich bekannten implantierbaren elektrischen multipoligen Verbindungsstruktur unter Bezugnahme auf die aus den Figuren 2a und b zu entnehmenden Illustrationen näher erläutert werden.

Figur 2a zeigt eine Cuff-Elektrodenanordnung 1, die als Wickelelektrode zur Umfassung um ein Nervenfaserbündel 2 ausgebildet ist. Zu Zwecken einer therapeutischen Stimulation des Nervenfaserbündels 2 sieht die Cuff-Elektrodenanordnung 1 eine Vielzahl einzelner Elektrodenflächen vor, die separat voneinander mit elektrischer Energie sowie Steuersignalen zu versorgen sind. Hierzu verläuft die Vielzahl einzelner elektrischer Zu- und Ableitungen 3 innerhalb eines flexiblen, flächig ausgebildeten Trägersubstrats 4, das als Polymerfolie ausgebildet ist. Die elektrischen Zu- und Ableitungen 3 enden in Form einer stirnseitigen Anschlussstruktur in Form von nebeneinanderliegend angeordneter, so genannter elektrisch leitender Mikroflexstrukturen 5, die in siehe Figur 2b detailliert dargestellt sind und die es einzeln elektrisch zu kontaktieren gilt.

Zu Zwecken der elektrischen Kontaktierung der Mikroflexstrukturen 5 dient in an sich bekannter Weise eine Keramikadapterplatte 6, auf der entsprechend der Anzahl und Anordnung der Mikroflexstrukturen 5 so genannte Mikroflexkontakte bzw. Mikroflexpads 7 angebracht sind, die in Kontakt mit den Mikroflexstrukturen 5 gebracht werden und elektrisch leitend jeweils einzeln mit an der Oberfläche der Keramikadapterplatte 6 angebrachten Elektrodenflächen 8 verbunden sind. Über Löt- oder Bondverbindungen 9 sind einzelne elektrische Drähte 10 eines elektrischen Kabels 11 mit den einzelnen als Lötpads ausgebildete Elektrodenflächen 8 verbunden. Selbstverständlich ist die gesamte in Figur 2b dargestellte, elektrische Verbindungsanordnung mit einem biokompatiblen Kunststoff möglichst vollständig fluiddicht umfasst.

Es liegt auf der Hand, dass der erforderliche Bauraum für die bekannte elektrische Verbindungsstruktur mit zunehmender Komplexität und Multipolarität der zu implantierenden Einheit zunimmt, wodurch auch die Patientenbelastung und -irritation in gleicher Weise zunimmt.

Die Druckschrift US 5,324,322 A offenbart eine implantierbare Elektrodenanordnung mit einem Wickelelektrodenteil in Art einer Cuff-Elektrode, die über einen Verbindungsabschnitt mit einer flächig ausgebildeten, multipoligen Verbindungsstruktur verbunden ist, auf der eine Anzahl von elektrischen Kontaktflächen aufgebracht ist. In einem Ausführungsbeispiel ist die flächige Verbindungsstruktur gleichsam dem Wickelelektrodenteil um eine Achse gewickelt. Im gewickelten Zustand nimmt die Verbindungsstruktur die Form eines Hohlzylinders an, der eine starre Bauform besitzt und zum Zwecke der intrakorporalen Verortung abschnittsweise um einen Nervenfaserstrang angelegt wird.

### Darstellung der Erfindung

Der Erfindung liegt die Aufgabe zugrunde eine implantierbare, elektrische, multipolige Verbindungsstruktur zwischen einem elektrischen Implantat und einer elektrischen Zu- und Ableitungsstruktur beispielsweise in Form eines multipoligen Kabels derart auszubilden, dass die patientenseitige Belastung gegenüber bekannten, gattungsgemäßen Verbindungsstrukturen signifikant reduziert werden soll und dies trotz großer Anzahl von einzelnen zu kontaktierenden, implantatseitigen elektrischen Zuleitungen. Die hierfür erforderlichen Maßnahmen sollen die Möglichkeit schaffen eine beliebig skalierbare Anzahl an elektrischen Zuleitungen über eine multipolige Verbindungsstruktur mit einem Implantat zu verbinden, ohne dass hierfür ein nennenswerter zusätzlicher Bauraum erforderlich wird.

Die Lösung der der Erfindung zugrundeliegenden Aufgabe ist im Anspruch 1 angegeben. Den Erfindungsgedanken in vorteilhafter Weise weiterbildende Merkmale sind Gegenstand der Unteransprüche sowie der weiteren Beschreibung, insbesondere unter Bezugnahme auf die Ausführungsbeispiele zu entnehmen.

Die implantierbare elektrische, multipolige Verbindungsstruktur zeichnet sich lösungsgemäß dadurch aus, dass zwischen dem Implantat und der Zu- und Ableitungsstruktur ein flexibles, folienartiges, elektrisch nicht leitendes, streifenförmiges Flächenelement angeordnet ist, mit wenigstens einer ersten Flächenelementoberfläche, an der eine Anzahl n ≥ 2 Elektroden angeordnet ist. Jede der n Elektroden ist mit einem elektrischen Verbindungsleiter verbunden, der zur Signalsteuerung und/oder Energieversorgung mit dem elektrischen Implantat verbunden ist. Die wenigstens n elektrischen Verbindungsleiter verlaufen zumindest bereichsweise innerhalb des Flächenelementes.

Die elektrische Zu- und Ableitungsstruktur umfasst wenigstens n elektrisch voneinander isolierte Drähte, die endseitig jeweils mit einer der n-Elektroden elektrisch verbunden sind, beispielsweise im Wege einer Bond- oder Lötverbindung. Zudem ist das streifenförmige Flächenelement, längs dessen wenigstens ersten Flächenelementoberfläche die n-Elektroden angeordnet sind und jeweils mit einer der n-Drähten verbunden sind, unter Ausbildung einer Helix um eine Wickelachse längs einer Mantelfläche eines virtuellen Zylinders mit einer geraden oder zumindest abschnittsweise gekrümmten Zylinderachse gewickelt.

Durch die lösungsgemäß vorgeschlagene Wicklung des streifenförmigen Flächenelementes um eine geradlinige oder zumindest abschnittsweise gekrümmte Zylinderachse, längs der sich vorzugsweise die elektrische Zu- und Ableitungsstruktur in Form eines Bündels von Drähten erstreckt, die zu einem Kabel zusammengefasst sind, ist kein zusätzlicher Bauraum erforderlich als jener, der für die Zu- und Ableitungsstruktur ohnehin benötigt wird. Durch die Flexibilität des folienartigen, elektrisch nicht leitenden Flächenelementes erfährt ein Patient keine nennenswerten irritierenden Wahrnehmungen, wie sie ansonsten von einer aus starrem Material gefertigten Keramikadapterplatte herrühren. Vielmehr vermag sich die lösungsgemäß ausgebildete multipolige, elastische Verbindungsstruktur an jeweilige intrakorporale räumliche Gegebenheiten individuell anzuschmiegen.

Eine besonders bevorzugte Ausführungsform sieht eine einstückige Bauweise bzw. Verbindung des Implantats mit dem flexiblen, folienartig, elektrisch nicht leitenden Flächenelement vor, in dem die Anzahl n an elektrischen Verbindungsleitern vollständig umfasst bzw. integriert ist und deren zu kontaktierenden Enden jeweils an einer Elektrode an wenigstens einer ersten Flächenelementoberfläche enden. Die einstückige Bauweise von Implantat und Flächenelement macht einerseits eine elektrische Kontaktanordnung zwischen Implantat und elektrische Verbindungsstruktur in Form des Flächenelementes überflüssig, andererseits kann unter Einsatz mikrosystemtechnischer Verfahrenstechniken eine nahezu beliebig skalierbare Anzahl von elektrischen Verbindungsleitern und jeweils deren Kontaktelektroden mit einem hohen Integrationsgrad im Bereich des Flächenelementes realisiert werden.

Aus Gründen einer einheitlichen und verfahrenstechnisch möglichst einfachen Realisierbarkeit der elektrischen Kontakte zwischen den auf der Flächenelementoberseite vorgesehenen Elektroden und den einzelnen elektrisch voneinander isolierten Drähten der elektrischen Zu- und Ableitungsstruktur, die im weiteren vereinfachend als Kabel bezeichnet wird, sind sämtliche n Elektroden auf einer gemeinsamen Flächenelementoberfläche angeordnet. Dies hat zudem den weiteren Vorteil, dass nach entsprechender Wicklung des Flächenelementes sämtliche mit dem Flächenelement verbundene Drähte einheitlich entweder mit den Elektroden an der Wickelachse zu- oder abgewandten Flächenelementoberfläche verbunden sind. Vorzugsweise erfolgt die Wicklung des Flächenelementes derart, so dass die mit Elektroden versehene Flächenelementoberfläche der Wickel- bzw. Zylinderachse radial zugewandt ist, so dass die mit den einzelnen Elektroden verbundenen Drähte zumindest teilweise vom Flächenelement im gewickelten Zustand radial umfasst werden.

Um eine möglichst große Anzahl an Elektroden auf dem Flächenelement vorzusehen, sieht eine Ausführungsform vor beide sich gegenüberliegenden Flächenelementoberflächen mit einer entsprechenden Anzahl an Elektroden zu belegen, die jeweils über einen, zumindest bereichsweise innerhalb des Flächenelementes verlaufenden elektrischen Verbindungsleiter mit dem elektrischen Implantat verbunden sind. Nicht notwendigerweise muss hierzu die Anzahl n Elektroden auf der ersten Flächenelementoberfläche mit einer Anzahl m Elektroden auf der gegenüberliegenden zweiten Flächenelementoberfläche übereinstimmen.

Die elektrische Verbindung der einzelnen Drähte des Kabels mit den jeweiligen Elektroden an der wenigstens ersten Flächenelementoberfläche erfolgt vorzugsweise mittels Löt-, Schweiß- oder Bondtechnik.

Zum Schutz der elektrischen Kontakte an den Elektroden längs des gewickelten Flächenelementes samt der von den einzelnen Elektroden wegführenden Drähten gegenüber dem intrakorporalen feuchten Milieu sieht eine bevorzugte Ausführungsform eine aus elektrisch isolierenden, biokompatiblen Material bestehende Umhüllung vor, die zumindest den Bereich des Flächenelementes vorzugsweise vollumfänglich umfasst. Die Umhüllung kann in Form einer schlauchförmigen Vergussmasse oder in Art eines flüssigkeitsdichten Schrumpfschlauches ausgebildet sein. Selbstverständlichen stehen dem Fachmann sämtliche ihm bekannte Verguss- bzw. Umhüllungstechniken zur Verfügung, um sämtlichen Medizinprodukt-spezifischen Anforderungen zu genügen.

Die streifenförmige Ausbildung des Flächenelementes, d.h. das Flächenelement zeichnet sich durch eine Längserstreckung aus, die sehr viel größer dimensioniert ist als die dem Flächenelement zuordenbare Streifenbreite, ermöglicht eine helikale Umwicklung des streifenförmigen Flächenelementes längs einer Mantelfläche eines virtuellen Zylinders. Durch die helikale Wickelform verfügt das streifenförmige Flächenelement im aufgewickelten Zustand über eine hohe Verformbarkeit bzw. Elastizität quer zur Wickelachse, sodass eine an die individuellen räumlichen, intrakorporalen Gegebenheiten beliebig anpassbare Raumform der Verbindungsstruktur ermöglicht wird, wodurch die Patientenbelastung auch und insbesondere in Fällen, in denen die Verbindungsstruktur eine große Vielzahl von elektrischen Kontakten besitzt, minimiert werden kann. Vorzugsweise ist die Vielzahl n Elektroden längs der radial nach innen orientierten Streifenoberseite angeordnet. Auf diese Weise wird die Vielzahl n jeweils von den einzelnen Elektroden wegführenden Drähte von dem spiralförmigen bzw. helikalen, streifenförmigen Flächenelement radial umfasst.

Um zu vermeiden, dass die elektrischen Kontaktstellen sämtlicher Drähte nach entsprechender Wicklung des Flächenelementes untereinander elektrische Kurzschlüsse ausbilden, sind die Kontaktstellen entsprechend mit Vergussmasse oder einer zusätzlichen Beschichtung gegenseitig isoliert. In einer bevorzugten Ausführungsform ist das zylinderförmige Volumen, das das um die Wickelachse aufgewickelte Flächenelement radial innen liegend umfasst, mit einem elastischen, elektrisch isolierenden, biokompatiblen Material gefüllt bzw. verfüllt.

Nicht notwendigerweise ist die lösungsgemäße multipolige Verbindungsstruktur in Form des flexiblen, folienartig ausgebildeten, elektrisch nicht leitenden Flächenelementes einstückig mit dem Implantat verbunden. Ebenso ist es denkbar, dass lediglich die zumindest bereichsweise innerhalb des Flächenelementes verlaufenden elektrischen Verbindungsleiter mittel- oder unmittelbar mit dem Implantat verbunden sind und in das zum Implantat separate Flächenelement einmünden.

Die lösungsgemäße implantierbare, elektrische, multipolige Verbindungsstruktur eignet sich in besonders vorteilhafter Weise zur elektrischen Signal- und Energieübertragung zwischen einem als Cuff-Elektrodenanordnung ausgebildeten Implantat und einer intra- oder extrakorporalen elektrischen Steuer- und Energieeinheit.

Eine an sich bekannte Cuff-Elektrodenanordnung ist bereits unter Bezugnahme auf Figur 2 beschrieben worden und wird in an sich bekannter Weise zur ortsselektiven Erfassung neuronaler elektrischer Signale, die sich längs wenigstens einer in einem Nervenfaserbündel enthaltenen Nervenfaser ausbreiten, sowie zur selektiven elektrischen Stimulation der wenigstens einen Nervenfaser eingesetzt. Die an sich bekannte Cuff-Elektrodenanordnung sieht ein biokompatibles Trägersubstrat vor, das zumindest einen Trägersubstratbereich aufweist, der um das Nervenfaserbündel manschettenartig anlegbar ist und eine im implantierten Zustand dem Nervenfaserbündel zugewandt orientierte geradzylinderförmige Trägersubstratoberfläche aufweist, an der eine Anzahl n Elektroden angebracht ist, die jeweils über innerhalb des biokompatiblen Trägersubstrates verlaufende elektrische Leitungen verbunden sind. Das lösungsgemäße Flächenelement ist einstückig mit dem biokompatiblen Trägersubstrat verbunden, so dass die elektrischen Leitungen innerhalb des Trägersubstrates nahtlos in den Bereich des Flächenelementes führen und dort mit dem n Elektroden an der Flächenelementoberfläche verbunden sind.

Die integrative Ausbildung des Trägersubstrates mit dem lösungsgemäß ausgebildeten Flächenelement bietet sich vor allem deshalb an, zumal sowohl das Trägersubstrat als auch das Flächenelement folienartig ausgebildet sind und aus einem flexiblen, elektrisch nicht leitenden Material, vorzugsweise aus einem Polymer, vorzugsweise Polyimid bestehen. In gleicher Weise wie der sich manschettenartig um das Nervenfaserbündel anliegende Trägersubstratbereich, der sich durch eine prozess- und materialtechnische Einprägung einer mechanischen Vorspannung eigenständig aufzuwickeln bzw. aufzurollen vermag, ist auch das über die n-Elektroden verfügende, vorzugsweise streifenförmig ausgebildete Flächenelement mit einer mechanischen Vorspannung zum Zwecke einer selbständigen Wicklung ausgeführt. Weitere Einzelheiten zur lösungsgemäßen implantierbaren elektrischen multipoligen Verbindungsstruktur sind unter Bezugnahme auf die nachfolgenden Figuren zu entnehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a bis d: Illustration einer lösungsgemäß ausgebildeten Cuff-Elektrodenanordnung in Form von Sequenzbilddarstellung zur Ausbildung derselben sowie
- Fig. 2a, b: an sich bekannte implantierbare Verbindungsstruktur gemäß Stand der Technik.

### Wege zur Ausführung der Erfindung, gewerbliche Verwendbarkeit

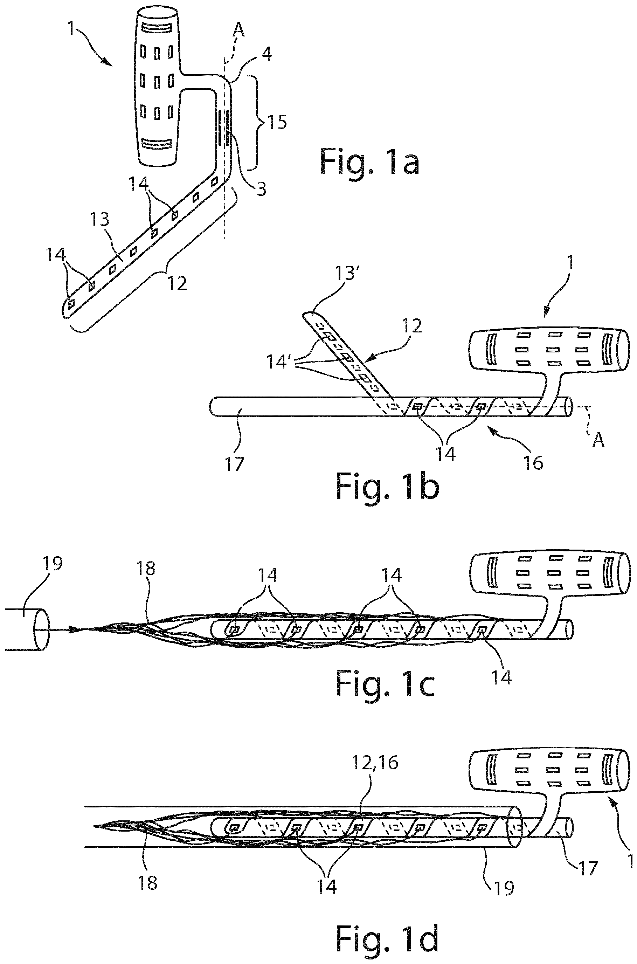

Figur 1a illustriert eine lösungsgemäß ausgebildete Cuff-Elektrodenanordnung 1, die einstückig mit einem streifenförmig ausgebildeten Flächenelement 12 verbunden ist. Das Flächenelement 12 sowie auch das Trägersubstrat 4 der Cuff-Elektrodenanordnung 1 sind aus einheitlichem Material, vorzugsweise Polyimid gefertigt und stellen eine flexible, elektrisch nicht leitende Folie dar. Innerhalb des Trägersubstrates 4 verlaufen in an sich bekannter Weise elektrische Leitungen 3, die einerseits mit Elektroden der Cuff- Elektrodenanordnung 1 und andererseits mit an der Flächenelementoberfläche 13 des streifenförmigen Flächenelementes 12 angebrachten, in Form von Löt- oder Bondpads ausgebildeten Elektroden 14 verbunden sind. Hierzu verlaufen die elektrischen Leitungen eingebettet innerhalb des Trägersubstrates 4 sowie längs des streifenförmigen Flächenelementes 12.

Das Trägersubstrat 4 weist einen länglich ausgebildeten Trägersubstratabschnitt 15 auf, der sich längs einer Achse A erstreckt. Die Achse A definiert die Wickelachse, um die das streifenförmig ausgebildete Flächenelement 12 unter Ausbildung einer helikalen Wickelstruktur umwickelt wird.

Figur 1b illustriert den Wickelvorgang, gemäß dem das streifenförmig ausgebildete Flächenelement 12 um die Wickelachse A unter Ausbildung einer Helix 16 umwickelt wird. Optional kann für den Wickelvorgang ein zylindrischer Hilfskörper 17 verwendet werden, der nach Abschluss des Wickelvorgangs entfernt oder als elektrisch isolierender Körper verbleibt. Vorzugsweise ist in diesem Fall der Hilfskörper 17 aus elastischem, elektrisch nicht leitendem, biokompatiblem Material gefertigt.

Vor oder nach dem Wickelvorgang werden die einzelnen Elektroden 14, die längs des streifenförmigen Flächenelementes 12 verteilt angeordnet sind, mit den Enden von elektrischen Drähten 18 verbunden, beispielsweise im Wege eines Löt- oder Bondvorganges, siehe Fig. 1c. Sämtliche Drähte 18 werden zu einem kompakten, flexiblen Kabel zusammengeführt. Dies erfolgt beispielsweise durch Aufschieben einer Umhüllung 19 in Längserstreckung zu der ausgebildeten Helix 16. Die Umhüllung 19 besteht aus einem biokompatiblen elektrisch isolierendem Material, beispielsweise in Form eines aufgequollenen Silikonschlauches.

Nach entsprechender Schrumpfung des Silikonschlauches und gegebenenfalls zusätzlicher innerer Verfüllung des Schlauchvolumens mit geeignetem Material, vorzugsweise PDMS ist ein fluiddichter Schutz sämtlicher innen liegender elektrischer Kontaktoberflächen hergestellt, siehe Figur 1d.

Die lösungsgemäß implantierbare elektrische multipolige Verbindungsstruktur verfügt im Unterschied zu der eingangs erläuterten, bekannten Verbindungstechnik über eine hohe Flexibilität und lediglich pro Draht über eine einzige elektrische Verbindungsstelle, die im Wege eines Bond-, Schweiß- oder Lötvorganges hergestellt werden kann. Ferner lässt sich die Anzahl der Elektroden und damit die Multipoligkeit der Verbindungsstruktur nahezu beliebig skalieren. So kann in diesem Zusammenhang das streifenförmig ausgebildete Flächenelement 12 zum einen entsprechend verlängert und zum anderen an beiden Flächenelementoberflächen mit Elektroden belegt werden, d.h. zusätzlich zu den n Elektroden 14 auf der ersten Flächenelementoberfläche 13 ist eine Anzahl m größer gleich zwei Elektroden (14') an einer der ersten Flächenelementoberfläche (13) abgewandt orientierten zweiten Flächenelementoberfläche (13') angebracht, siehe Fig. 1b.

### Bezugszeichenliste

- 1: Cuff-Elektrodenanordnung
- 2: Nervenfaserbündel
- 3: Elektrische Leitungen
- 4: Biokompatibles Trägersubstrat
- 5: Mikroflexstrukturen
- 6: Keramikadapterplatte
- 7: Mikroflexkontakte, Mikroflexpads
- 8: Elektrodenflächen
- 9: Lötstelle
- 10: Elektrischer Versorgungsdraht
- 11: Kabel
- 12: Streifenförmiges Flächenelement
- 13: erste Flächenelementoberfläche
- 13': zweite Flächenelementoberfläche
- 14, 14': Elektrode
- 15: Trägersubstratabschnitt
- 16: Helix
- 17: Hilfskörper
- 18: Drähte
- 19: Umhüllung
- A: Wickelachse

## Patentansprüche

1. Implantierbare elektrische, multipolige Verbindungsstruktur zwischen einem elektrischen Implantat (1) und einer elektrischen Zu- und Ableitungsstruktur, wobei zwischen dem Implantat (1) und der Zu- und Ableitungsstruktur (3) ein flexibles, folienartiges, elektrisch nicht leitendes, streifenförmig ausgebildetes Flächenelement (12) angeordnet ist, mit wenigstens einer ersten Flächenelementoberfläche (13), **dadurch gekennzeichnet, dass** an der Flächenelementoberfläche (13) eine Anzahl n größer gleich zwei Elektroden (14) angeordnet ist, die jeweils über zumindest bereichsweise innerhalb des Flächenelementes (12) verlaufende, elektrische Verbindungsleiter (3) mit dem elektrischen Implantat (1) verbunden sind,
dass die elektrische Zu- und Ableitungsstruktur (3) wenigstens n elektrisch voneinander isolierte Drähte (18) umfasst, die jeweils mit einer der n Elektroden (14) elektrisch verbunden sind, und
dass das streifenförmige Flächenelement (12) unter Ausbildung einer Helix um eine Wickelachse (A) längs einer Mantelfläche eines virtuellen Zylinders mit gerader oder zumindest abschnittsweise gekrümmter Zylinderachse gewickelt ist.

2. Verbindungsstruktur nach Anspruch 1,
**dadurch gekennzeichnet, dass** die wenigstens eine Flächenelementoberfläche (13) der Mantelfläche des virtuellen Zylinders radial zu- oder abgewandt ist.

3. Verbindungsstruktur nach Anspruch 1 oder 2,
**dadurch gekennzeichnet, dass** eine zweite Flächenelementoberfläche (13') vorgesehen ist, längs der eine Anzahl m größer gleich 2 Elektroden (14') angeordnet ist, die jeweils über zumindest bereichsweise innerhalb des Flächenelementes (12) verlaufende, elektrische Verbindungsleiter mit dem elektrischen Implantat (1) verbunden sind, und
dass die zweite Flächenelementoberfläche (13') zur ersten Flächenelementoberfläche (13) abgewandt orientiert ist.

4. Verbindungsstruktur nach einem der Ansprüche 1 bis 3,
**dadurch gekennzeichnet, dass** um das, um die Wickelachse (A) gewickelte streifenförmige Flächenelement (12) samt der mit den Elektroden (14) elektrisch verbundenen Drähte (18), die längs der Wickelachse (A) orientiert sind, eine schlauchförmige, aus elektrisch isolierendem, biokompatiblen Material bestehende Umhüllung (19) angebracht ist.

5. Verbindungsstruktur nach einem der Ansprüche 1 bis 4,
**dadurch gekennzeichnet, dass** das um die Wickelachse (A) gewickelte streifenförmige Flächenelement (12) ein zylinderförmiges Volumen umfasst, das mit einem elastischen, elektrisch isolierenden, biokompatiblen Material gefüllt ist.

6. Verbindungsstruktur nach einem der Ansprüche 1 bis 5,
**dadurch gekennzeichnet, dass** das Implantat (1) und das flexible, folienartige, elektrisch nicht leitende streifenförmige Flächenelement (12) einstückig verbunden sind.

7. Anordnung mit der Verbindungsstruktur nach einem der Ansprüche 1 bis 6 zur elektrischen Signal- und Energieübertragung zwischen einem als Cuff-Elektrodenanordnung ausgebildeten Implantat (1) und einer intra- oder extrakorporalen elektrischen Steuer- und Energieeinheit (20).

8. Anordnung nach Anspruch 7,
**dadurch gekennzeichnet, dass** die Cuff-Elektrodenanordnung (1) zur ortsselektiven Erfassung neuronaler elektrischer Signale, die sich längs wenigstens einer in einem Nervenfaserbündel enthaltenden Nervenfaser ausbreiten, sowie zur selektiven elektrischen Stimulation der wenigstens einen Nervenfaser ausgebildet ist, die ein biokompatibles Trägersubstrat vorsieht, das zumindest einen Trägersubstratbereich aufweist, der um das Nervenfaserbündel manschettenartig anlegbar ist und eine im implantierten Zustand dem Nervenfaserbündel zugewandt orientierte, geradzylinderförmige Trägersubstratoberfläche aufweist, an der eine Anzahl n Elektroden angebracht ist,
dass die n Elektroden jeweils über innerhalb des biokompatiblen Trägersubstrats angeordneten elektrischen Leitungen verbunden sind, und
dass das flexible, folienartige, elektrisch nicht leitende Flächenelement der Verbindungsstruktur einstückig mit dem biokompatiblen Trägersubstrat verbunden ist und die elektrischen Leitungen mit den n Elektroden an der Flächenelementoberfläche verbunden sind.

9. Anordnung nach Anspruch 8,
**dadurch gekennzeichnet, dass** das Trägersubstrat und das flexible, folienartige, elektrisch nicht leitende Flächenelement aus Polyimid gefertigt sind.

## Claims

1. An implantable electric multi-pole connection structure between an electric implant (1) and an electric feed and discharge structure, wherein a flexible, film-like, electrically non-conductive, strip-shaped surface element (12) is arranged between the implant (1) and the feed and discharge structure (3), with at least a first surface (13) of the surface element, **characterised in that** on the first surface (13) of the surface element a number of electrodes (14) n, greater than or equal to two, is arranged, each of which is connected to the electric implant (1) via electric connection conductors (3), running at least in parts inside the surface element (12),
**in that** the electric feed and discharge structure (3) comprises at least n wires (18), electrically insulated from each other, each of which is electrically connected to one of the n electrodes (14); and
**in that** the strip-shaped surface element (12) is wound around a winding axis (A), which adopts the shape of a helix, along a cover surface of a virtual cylinder provided with a cylinder axis, which is straight or curved at least in sections.

2. The connection structure in accordance with claim 1, **characterised in that**, the at least one surface (13) of the surface element radially faces towards or away from the cover surface of the virtual cylinder.

3. The connection structure in accordance with claim 1 or 2, **characterised in that**, a second surface (13') of the surface element is provided, along which is arranged a number of electrodes (14') m, greater than or equal to 2, each of which is connected to the electric implant (1) via electric connection conductors running at least in parts inside the surface element (12), and
**in that** the second surface (13') of the surface element is oriented away from the first surface (13) of the surface element.

4. The connection structure in accordance with one of the claims 1 to 3, **characterised in that**, a tubular sheath (19) consisting of an electrically insulating biocompatible material is arranged around the strip-shaped surface element (12) wound around the winding axis (A), together with the wires (18) electrically connected to the electrodes (14), and oriented along the winding axis (A).

5. The connection structure in accordance with one of the claims 1 to 4, **characterised in that**, the strip-shaped surface element (12) wound around the winding axis (A) comprises a cylindrical volume, which is filled with an elastic, electrically insulating, biocompatible material.

6. The connection structure in accordance with one of the claims 1 to 5, **characterised in that**, the implant (1) and the flexible, film-like, electrically non-conductive strip-shaped surface element (12) are integratively connected.

7. An arrangement with the connection structure in accordance with one of the claims 1 to 6, for purposes of electric signal and power transmission between an implant (1) designed as a cuff electrode arrangement and an intra- or extra-corporeal electric control and power unit (20).

8. The arrangement in accordance with claim 7, **characterised in that**, the cuff electrode arrangement (1) is designed for purposes of location-selective recording of neuronal electric signals propagating along at least one nerve fibre contained in a nerve fibre bundle, and for purposes of selective electric stimulation of the at least one nerve fibre; it provides a biocompatible supporting substrate, which has at least one supporting substrate region, which can be placed around the nerve fibre bundle in the form of a cuff, and has a straight cylindrical supporting substrate surface, which in the implanted state is oriented towards the nerve fibre bundle, and to which a number of electrodes n is attached,
**in that** the n electrodes are each connected via electric lines arranged inside the biocompatible supporting substrate, and
**in that** the flexible, film-like, electrically non-conductive surface element of the connection structure is integratively connected to the biocompatible supporting substrate, and the electric lines are connected to the n electrodes on the surface of the surface element.

9. The arrangement in accordance with claim 8, **characterised in that**, the supporting substrate and the flexible, film-like, electrically non-conductive surface element are made of a polyimide.

## Revendications

1. Structure de liaison implantable, électrique, multipolaire entre un implant électrique (1) et une structure d'alimentation et de dérivation électrique, sachant qu'entre l'implant (1) et la structure d'alimentation et de dérivation (3) est disposé un élément de surface (12) constitué en forme de bande, flexible, de type feuille, non électriquement conducteur, avec au moins une première surface d'élément de surface (13), **caractérisée en ce que** sur la surface d'élément de surface (13) est disposé un nombre 'n' plus grand ou égal à deux électrodes (14), qui sont reliées respectivement à l'implant électrique (1) par des conducteurs de liaison (3) électriques passant au moins par zone à l'intérieur de l'élément de surface (12),
**en ce que** la structure d'alimentation et de dérivation électrique (3) comprend au moins 'n' fils (18) électriquement isolés les uns des autres, qui sont respectivement reliés électriquement à une des 'n' électrodes (14), et
**en ce que** l'élément de surface en forme de bande (12) est enroulé en formant une hélice autour d'un axe d'enroulement (A) le long d'une surface d'enveloppe d'un cylindre virtuel avec un axe de cylindre droit ou incurvé au moins par section.

2. Structure de liaison selon la revendication 1,
**caractérisée en ce qu'**au moins une surface d'élément de surface (13) est radialement tournée ou opposée à la surface d'enveloppe du cylindre virtuel.

3. Structure de liaison selon la revendication 1 ou 2, **caractérisée en ce qu'**une deuxième surface d'élément de surface (13') est prévue le long de laquelle un nombre 'm' plus grand ou égal à 2 électrodes (14') est disposé, qui sont respectivement reliées à l'implant électrique (1) par des conducteurs de liaison électriques passant au moins par zone à l'intérieur de l'élément de surface (12) et
**en ce que** la deuxième surface d'élément de surface (13') est orientée opposée à la première surface d'élément de surface (13).

4. Structure de liaison selon l'une quelconque des revendications 1 à 3,
**caractérisée en ce qu'**une enveloppe (19) de forme tubulaire, composée d'un matériau biocompatible, électriquement isolant est disposée autour de l'élément de surface (12) en forme de bande enroulé autour de l'axe d'enroulement (A) avec les fils (18) reliés électriquement aux électrodes (14), qui sont orientés le long de l'axe d'enroulement (A).

5. Structure de liaison selon l'une quelconque des revendications 1 à 4,
**caractérisée en ce que** l'élément de surface (12) en forme de bande enroulé autour de l'axe d'enroulement (A) comprend un volume cylindrique, qui est rempli d'un matériau élastique, biocompatible, électriquement isolant.

6. Structure de liaison selon l'une quelconque des revendications 1 à 5,
**caractérisée en ce que** l'implant (1) et l'élément de surface flexible, de type feuille, non électriquement conducteur, en forme de bande (12) sont reliés en une seule pièce.

7. Système avec la structure de liaison selon l'une quelconque des revendications 1 à 6 pour la transmission électrique de signaux et d'énergie entre un implant (1) constitué comme un système d'électrodes à manchon et une unité de commande et d'énergie électrique intra ou extracorporelle (20).

8. Système selon la revendication 7,
**caractérisé en ce que** le système d'électrodes à manchon (1) est constitué pour la saisie localement sélective de signaux neuronaux électriques, qui s'étendent le long d'au moins une fibre nerveuse contenue dans un faisceau de fibres nerveuses, ainsi que pour la simulation électrique sélective d'au moins une fibre nerveuse, qui prévoit un substrat de support biocompatible, qui comporte au moins une zone de substrat de support, qui peut être posée comme un manchon autour du faisceau de fibres nerveuses et une surface de substrat de support de forme cylindrique droite, orientée tournée à l'état implanté vers le faisceau de fibres nerveuses, sur laquelle est disposé un nombre 'n' d'électrodes,
**en ce que** les 'n' électrodes sont respectivement reliées par des conducteurs électriques disposés à l'intérieur du substrat de support biocompatible, et
**en ce que** l'élément de surface flexible, de type feuille, non électriquement conducteur de la structure de liaison est relié en une seule pièce au substrat de support biocompatible et les conducteurs électriques sont reliés aux 'n' électrodes sur la surface d'élément de surface.

9. Système selon la revendication 8,
**caractérisée en ce que** le substrat de support et l'élément de surface flexible, du type feuille, non électriquement conducteur sont fabriqués en polyimide.
